(19) 

Europäisches
Patentamt
European
Patent Office
Office européen
des brevets

(11) **EP 2 041 636 B1**

(12) **EUROPEAN PATENT SPECIFICATION**

(45) Date of publication and mention
of the grant of the patent:
**18.11.2009 Bulletin 2009/47**

(51) Int Cl.:
***G05D 1/02*** (2006.01)

(21) Application number: **07825363.0**

(86) International application number:
**PCT/IB2007/003059**

(22) Date of filing: **06.07.2007**

(87) International publication number:
**WO 2008/007225 (17.01.2008 Gazette 2008/03)**

(54) **METHOD AND SYSTEMS FOR LOCATING A SOURCE OF PARTICLE, MOLECULE, OR FRAGMENT OF MOLECULE USING THEIR RECEPTION RATE**

VERFAHREN UND SYSTEME ZUR LOKALISIERUNG EINER TEILCHEN-, MOLEKÜL- ODER MOLEKÜLFRAGMENTQUELLE MIT HILFE IHRER EMPFANGSRATE

PROCÉDÉS ET SYTÈMES DE LA LOCALISATION DE LA SOURCE D'UNE PARTICULE ,D'UNE MOLECULE OU D'UNE PARTIE D'UNE MOLECULE EN UTILISANT LEUR TAUX DE RÉCEPTION.

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **07.07.2006 US 481944**
**07.07.2006 EP 06291133**

(43) Date of publication of application:
**01.04.2009 Bulletin 2009/14**

(73) Proprietors:
• **INSTITUT PASTEUR**
**75015 Paris (FR)**
• **Vergassola, Massimo**
**75017 Paris (FR)**
• **Shraiman, Boris**
**Goleta, CA 93117 (US)**
• **Villermaux, Emmanuel**
**13006 Marseille (FR)**

(72) Inventors:
• **VERGASSOLA, Massimo**
**F-75017 Paris (FR)**

• **SHRAIMAN, Boris**
**Goleta, CA 93117 (US)**
• **VILLERMAUX, Emmanuel**
**F-13006 Marseille (FR)**

(74) Representative: **Santarelli**
**14, avenue de la Grande Armée**
**Boîte Postale 237**
**75822 Paris Cedex 17 (FR)**

(56) References cited:
• **CAMILLI R ET AL: "Integrating in-situ chemical sampling with auv control systems" OCEANS '04. MTTS/IEEE TECHNO-OCEAN '04 KOBE, JAPAN NOV. 9-12, 2004, PISCATAWAY, NJ, USA,IEEE, 9 November 2004 (2004-11-09), pages 101-109, XP010775989 ISBN: 0-7803-8669-8**
• **ADAM T HAYES ET AL: "Distributed Odor Source Localization" IEEE SENSORS JOURNAL, IEEE SERVICE CENTER, NEW YORK, NY, US, vol. 2, no. 3, June 2002 (2002-06), XP011065655 ISSN: 1530-437X**

**Description**

**Field of the Invention**

The present invention relates generally to a method and systems for locating particle or molecule sources and more specifically to a method and systems for locating the source of diffused particles or molecules using their reception rate.

**Background of the Invention**

Even if locating particle sources like odorous substances is a common task for animals, for example when searching for food, machines face difficulties in handling such problem despite the needs e.g., locating drugs, chemical leaks, explosive, and mines. Semiconductor gas-sensors are able to detect the presence or not of specific odorous substances and to determine their concentration however, locating the sources has to take into account the environment and particularly the air or liquid flow in which the odorous substance diffuses in a chaotic way.

Chemotactic bacteria lack a sense of position and their motion is perturbed by thermal noise, yet guided by the local gradient in nutrient concentration they can find its source. Macroscopic searchers endowed with a sense of direction and position often face a different problem: lack of local clues pointing towards the location of the target. For example, animals sensing odors in air or water detect them only intermittently as patches of odor sweep by, carried by winds and currents. Because of randomness of the advection and mixing process, local gradients of odor intensity do not point to the source and the searcher must devise a strategy of movement based upon sporadic cues and partial information.

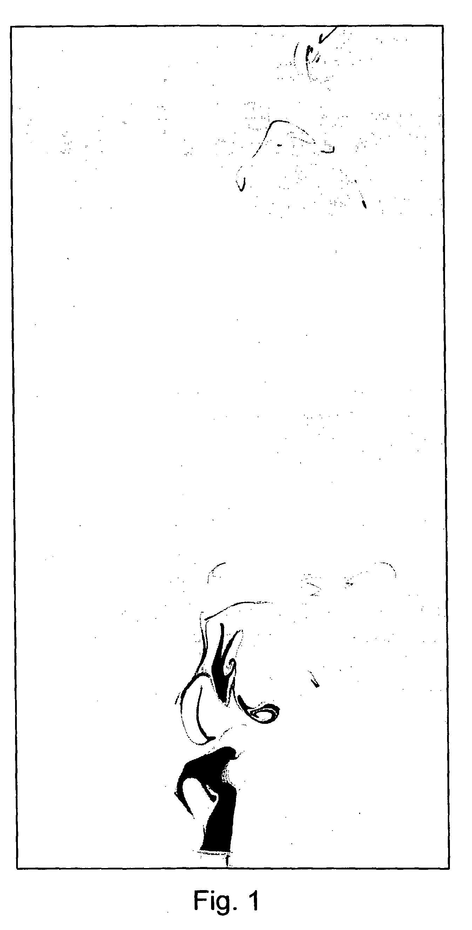

Like chemotactic bacteria, most of the robots equipped with odor sensor use the local concentration gradient of an odorous substance to determine locally the direction of its source, referred to as chemotactic search strategy. However, chemotactic search strategies based on local concentration gradients require concentration to be sufficiently high so that its average difference measured at two nearby locations is larger than typical fluctuations. The signal-to-noise ratio depends of course on the averaging time and might be improved by waiting. However, average concentration may be decaying rapidly e.g., exponentially, with the distance away from the source and in this weak signal-to-noise (dilute) case waiting becomes worse than exploratory motion. As an illustration, figure 1 depicts an example of an environment where odorous substance is diffused within the atmosphere and where odorous substance concentration can not be used locally to determine the odorous substance source.

Chemotaxis requires a reliable measurement of local gradients. This is not feasible for robots located far away from the source and severely limits the range of application of automated source localization by robots. Existing chemoatic robots might take several minutes to locate a few meters away.

Therefore, there is a need to provide a method and systems for solving the challenge of searching particle or molecule sources in dilute environment e.g., for the design of sniffers or robots that track chemicals emitted by drugs, chemical leaks, explosives and mines.

In the paper entitled "Integrating in-situ chemical sampling with AUV control systems", CAMILLI R ET AL, OCEANS'04. MTTS/IEEE TECHNO-OCEAN '04 KOBE, JAPAN NOV. 9-12, 2004, PISCATAWAY, NJ, USA, IEEE, 9 November 2004 (2004-11-09), pages 101-109, the authors explores conceptual frameworks for integrating payload sensors in an autonomous underwater vehicle in various degrees of real-time data assimilation and adaptive operation. Several of the challenges to coupling chemical sensor payloads in closed-loop architecture with acoustic, visual and navigation control systems are examined. Specific examples are provided as to how information sharing and coupled decision making processes may improve payload data interpretation and validation as well as increase the overall efficiency of AUV mission strategies. Results are discussed and a collection of general principles is suggested for integration of biological and chemical sensors as payload with active feedback aboard AUVs. The authors conclude with suggestions for possible scientific applications that can be addressed using levels of technology presently available as well as how incremental advancements in AUV payload integration will present profound new opportunities to explore and understand our world.

**Summary of the Invention**

Thus, it is a broad object of the invention to remedy the shortcomings of the prior art as described here above.

It is another object of the present invention to provide a method and systems for locating a particle or molecule source in diffuse environment.

It is another object of the invention to provide a method and systems for locating a particle or molecule source using particle or molecule reception rate.

It is still another object of the invention to provide a method and systems for locating a particle or molecule source by determining the probability for each point of the search space to be the particle or molecule source and by continuously updating these probabilities.

It is still another object of the invention to provide a method and systems for determining the direction of a particle or molecule source using the entropy of the probability of each search space point to be the particle or molecule source.

It is a particular object of the invention to provide a method and systems for determining the location of a particle or molecule source using the entropy of the probability of each search space point to be the particle or molecule source.

The accomplishment of these and other related objects is achieved by a method for locating a source of particles, molecules, or fragments of molecules in a search space using a mobile sensor adapted to detect the presence of such particle, molecule, or fragment of particle, the method comprising the steps of,

- determining the particle, molecule, or fragment of molecule diffusion parameters in the search space;

- designing a lattice on the search space;

- determining if at least one particle, molecule, or fragment of molecule is detected by the sensor;

- computing a probability for each node of the search space lattice to be the source of the particles, molecules, or fragments of molecules according to the determined particle, molecule, or fragment of molecule diffusion parameters and to the detected particles, molecules, or fragments of molecules;

- evaluating a movement of the sensor according to the computed probabilities.

Further embodiments of the invention are provided in the appended dependent claims.

Further advantages of the present invention will become apparent to the ones skilled in the art upon examination of the drawings and detailed description. It is intended that any additional advantages be incorporated herein.

## Brief Description of the Drawings

Figure 1 illustrates an example of an environment where odorous substance is diffused within the atmosphere and where odorous substance concentration can not be used locally to determine the odorous substance source.

Figure 2 shows an example of a computer architecture adapted to implement the method of the invention.

Figure 3 depicts the main steps of the algorithm according to the invention.

Figure 4 depicts the main steps of evaluating the posterior probability of each node of the search space lattice.

Figure 5 shows the main steps of evaluating the next optimal movement of the particle or molecule sensor according to the posterior probability of each node of the search space lattice.

Figure 6, comprising figures 6a and 6b, presents the result of a numerical simulation of particle source localization, in the absence of wind and in the presence of wind, respectively.

Figure 7 depicts the quantitative characterization of search algorithm according to the invention.

Figure 8 presents the path generated when localizing a particle source in a simulation utilizing experimental measurements of dye concentration in a turbulent flow.

## Detailed Description of the Preferred Embodiment

An example of organisms performing olfactory search in a dilute limit is provided by moths which use pheromones to locate their mates. Moths are known to proceed upwind via counterturning patterns of extended ("casting") or limited ("zigzagging") crosswind width thought to correlate with low and high rates of odor detection. In the dilute limit the searcher detects odor in a sporadic sequence of distinct events arising from its encounters with patches of fluid (or air) where turbulent mixing has failed to dissipate the advected odor down to the level below detectability threshold.

These detection events, or "hits", are separated by wide "voids" with no detectible signal. Because the probability of odor encounter depends on the distance from the source, the set of encounters which occurred at times $\{t_i\}$ along the search trajectory r(t) carries information about the source location.

According to the invention there is provided a method and systems based upon the rate of acquisition of information on particle or molecule source location, as estimated by the searcher, wherein the strategy of motion maximizes the expected rate of information gain. By particle or molecule source, one should understand any types of particles or molecules, comprising biological molecules such as protein or protein fragments, particularly pathogens. The efficiency of the method and system in reaching the source has been demonstrated and quantified computationally using a model of odor plume propagation as well as experimental data on mixing flows. An example of application the search algorithm is relevant to is the design of olfactory robots with applications to detection of chemical leaks and explosives however, the method of the invention can be applied more broadly in the context of searching with sparse information and provides a framework for quantitative understanding of the balance between the competing "exploration" and "exploitation" behaviors in learning processes.

For sake of illustration, the following description concerns a robot searching for an odorous substance source. By robot, one should understand a system comprising,

- a detector of particles or molecules (of the type emitted by the source to be localized);
- a system allowing its mobility; and,
- a processor to implement the calculations called by the decision protocol.

If detection in the presence of wind is foreseen, an anemometer to detect the mean direction of winds and/or currents is preferably embedded within the robot or, alternatively, the wind characteristics are transmitted to the robot.

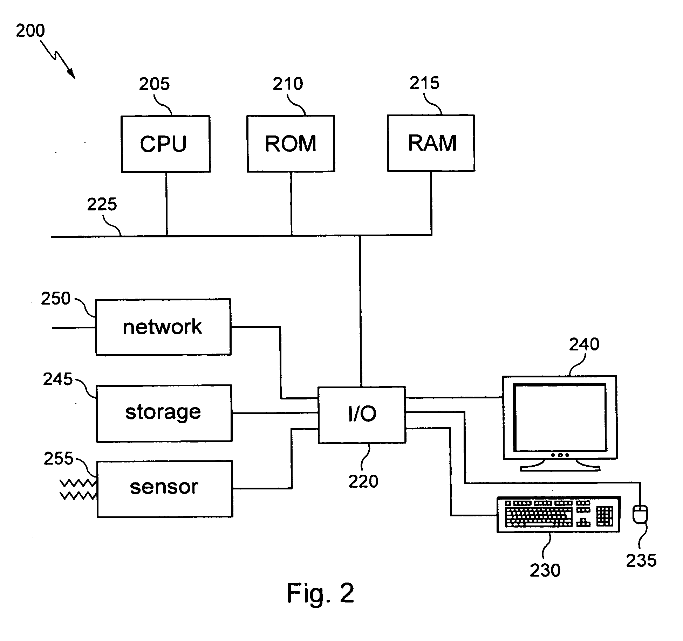

In a specific embodiment, the method of the invention is implemented within a standard computer architecture embedded within the robot. Figure 2 shows an example of a computer architecture adapted to implement the method of the invention. Figure 2 illustrates a block diagram of a generic computer device, handheld device, or any kind of computer device, generally referred to as computer 200, in which the present invention can be implemented. The system has a central processing unit (CPU) 205, a Read-Only Memory (ROM) ) 210, a Random Access Memory (RAM) 215, and an I/O subsystem 220, all of them being connected to a system bus 225. The I/O subsystem 220 may include one or more controllers for input/output devices such as keyboard 230, cursor control device 235, display device 240, mass storage device 245, and network interface 250. Depending upon the application of the system 200, one or more further I/O devices may be connected to the I/O subsystem 220. Typically, the hardware system 200 is controlled by an operating system that can be stored in ROM 210 or in mass storage device 245, which in turn controls various tools and applications that are generally loaded in RAM 215. At least one sensor 255 is connected to the I/O subsystem 220 for detecting the particle or molecule that source is searched. The system may include further sensors to detect other type of particles or molecules, or to analyze the environment e.g., detecting obstacles.

Alternatively, a robot comprising a particle or molecule sensor can be connected to a second computer or server using a wire or a wireless connection so that all the complex computation tasks can be done by the second computer or server. In such case, the computer embedded within the robot is used to control the robot, to transmit information relative to detected particles or molecules to the computer or server, and to receive instructions from the second computer or server concerning robot movement.

Example of algorithm according to the invention

The method of the invention is based upon the evaluation of the probability, for each point of the searching space, to be the particle or molecule source. Knowing such probability field, each possible movement of the robot is analyzed so as to maximize the diminution of the probability field entropy. To that end, the search space is divided into sub-spaces according to a predetermined grid where the probability is estimated for each node.

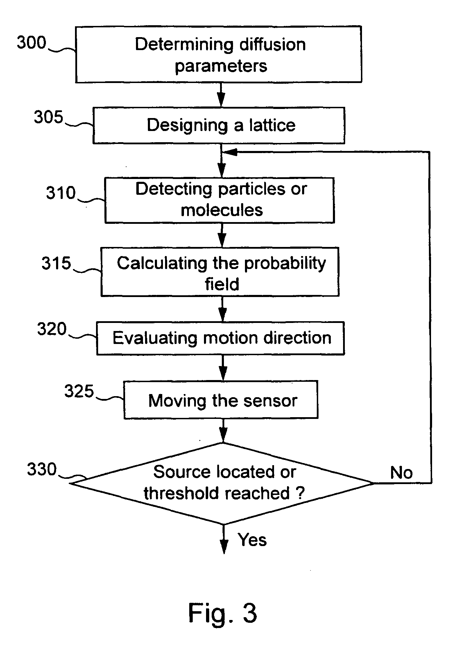

The main steps of the algorithm according to the invention are depicted on figure 3 and consists in,

- determining the particle or molecule diffusion parameters in the search space (step 300);
- designing a lattice on the search space (step 305);
- detecting in the search space, with or without wind or current, of one or several types of particles or molecules characteristic of the source to be localized, using detectors or sensors, preferably carried by a robot (step 310);
- calculating a probability field for the location of the source on the basis of the history of detections i.e., updating the probability for each node of the search space lattice to be the particle or molecule source, using the particle or molecule diffusion parameters (step 315);
- evaluating the direction of motion of the detector or sensor, or of the robot carrying the sensor, (step 320). The extension of the region of space where the source has a sizable chance of being located is estimated according to the probability distribution constructed at the previous step. The choice of the direction of motion is then dictated by the principle that the extension of the region (and thus the uncertainty on the location of the source) should reduce as rapidly as possible. This is quantified by the reduction of probability field entropy;

- moving the robot, or at least the detectors or sensors according to the direction of motion evaluated in the previous step (step 325); and,
- repeating the last four steps until the particle source is located or until a predetermined parameter reaches a predetermined threshold (step 330).

As mentioned above, one of the advantages of the algorithm according to the invention, as compared to most of the existing chemotactic strategies, is related to its capacity to deal with an uncertain environment where gradients cannot be reliably measured. No wind or particular reference points and/or directions are needed for the rapid location of the source even if they might be exploited if available. Note also that if several types of particles or molecules are detectable and characteristic of the spectrum of emission of the source, they can be combined according to the information that they provide about the location of the source. The process of source location will consequently be sped up.

Determination of the particle or molecule diffusion parameters

The rate of encounters between a spherical particle or molecule of radius a and particles or molecules diffusing with effective diffusivity D is given by the following classical Smoluchowski's expression,

$$J(r) = 4\pi Dac(r) \qquad (1)$$

where c(r) is the concentration of particles or molecules at position r, formally r(t) (for sake of clarity, the time index is not systemically written).

For a reliable assessment of concentration, one collects local detection events over time $T_{int}$. The average number of detection events will then be $J(r)T_{int}$. Typical fluctuations are of the order of the square root of the mean. The condition for the signal to emerge out of the noise reads then,

$$\sqrt{T_{int} Dac(r)} \gg 1 \qquad (2)$$

Reliable measurements of concentration gradients require the difference in counts across the interval of measurement to be above the noise level. The corresponding conditions read,

$$\left(vT_{int}\frac{dc}{dr}\right)T_{int} Da \gg \sqrt{DcT_{int}a} \; ; \qquad vT_{int}\frac{dLogc}{dr} \ll 1 \qquad (3)$$

Here, v is the velocity of the searcher and dc/dr is the concentration gradient. The first inequality in the previous relation gives the condition that the signal-to-noise ratio for the difference in the number of hits experienced by the searcher across the integration time $T_{int}$ be larger than unity. The second inequality is the requirement of locality, i.e. that the change in concentration across the distance spanned during $T_{int}$ be small compared to the concentration itself. Assuming an exponentially decaying concentration exp(-r/λ)), reliable integration time $T_{int}$ scales as exp(r/3λ) where λ represents the typical length traveled by particles or molecules away from the source.

A first reasonable statistics of odor encounters in a turbulent flow is provided by a model where detectable particles are emitted by the source at rate R, have a finite lifetime τ, propagate with isotropic effective diffusivity D and are advected by a mean current or wind V. It should be understood that other propagation models can be used, there are no particular restrictions on their nature.

The field of mean stationary concentration $c(r|r_0)$ generated at position r by a source located at position $r_0$, will satisfy the following advection-diffusion equation,

$$0 = V\nabla_y c(r \mid r_0) + V\Delta c(r \mid r_0) - \frac{1}{\tau}(r \mid r_0) + R\delta(r_0) \qquad (4)$$

where the wind has been taken to blow in the negative y-direction, $\delta(r_0)$ representing the source term. One should notice that V is the mean velocity and that the instantaneous velocity fluctuates both in direction and amplitude due to the noise term described by the effective diffusivity term D characterizing the sum of both the turbulent diffusivity and the (usually much smaller) particle or molecular diffusivity. Both in two and three dimensions, equation (4) admits an analytical solution. In 2D, the solution reads as follow,

$$c(r \mid r_0) = \frac{R}{2\pi D} e^{\frac{-(y-y_0)V}{2D}} K_0\left(\frac{|r-r_0|}{\lambda}\right) \qquad (5)$$

wherein $(y-y_0)$ represents where the wind was taken to blow in the y direction, $y_0$ is the y-coordinate of the source, and wherein,

$$\lambda = \sqrt{\frac{D\tau}{1 + \frac{V^2\tau}{4D}}} \qquad (6)$$

$K_0$ being the modified Bessel function of order zero. A similar expression is derived in three dimensions as:

$$c(r \mid r_0) = \frac{R}{4\pi Dr} e^{\frac{-(y-y_0)V}{2D}} e^{-\frac{r}{\lambda}} \qquad (7)$$

where $\lambda$ is given by the relation 6.

A spherical object of small linear size a moving into such media will experience a series of encounters at rates $R(r|r_0)$ given by the Smoluchowski's arguments. In three dimensions, the expression derives from relations 1 and 7.

In two dimensions, the time of return to a given location for a diffusive particle is known to have a logarithmic divergence. The logarithmic divergence is regularized by the presence of a finite lifetime of detected particles so that relation 1 takes the following form,

$$R(r \mid r_0) = \frac{2\pi Dc(r \mid r_0)}{\ln\left(\frac{\lambda}{a}\right)} = \frac{R}{\ln\left(\frac{\lambda}{a}\right)} e^{\frac{(y_0-y)V}{2D}} K_0\left(\frac{|r-r_0|}{\lambda}\right) \qquad (8)$$

with the concentration profile given by relation 7, where $R(r|r_0)$ represents the rate at which particles or molecules are received at point r from particle or molecule source located in $r_0$.

As a consequence, in three dimensions, the rate at which particles are received at point r from particle source located in $r_0$ can be expressed as follow,

$$R(r \mid r_0) = \frac{aR}{|r - r_0|} e^{\frac{-(r-r_0)}{\lambda}} e^{\frac{(y_0-y)V}{2D}} \qquad (9)$$

The properties of propagation of the cues through the medium and their frequency of emission have hitherto been considered as known. Robots designed to detect patches of odors - sniffers - are typically endowed with sensors measuring the time-trace of the wind that provide them with relatively reliable estimates of mean quantities such as the mean wind and the Root Mean Square (RMS) $V_{rms}$ of velocity fluctuations. The sporadic nature of the signal detected by sniffers comes from the fact that odors are typically transported by strongly turbulent fields that efficiently mix and make the patches spread out and decay in intensity with time. Parameters of turbulent flows are notoriously hard to estimate but the major advantage here is that searches according to the invention are not maximum likelihood strategies and are more robust to errors and incomplete information. Sophisticated modelling of the turbulent medium is therefore not crucial (and it is hard to imagine that birds and moths rely on fine-tuning of parameters in their searches). The most effective approach to parameter estimation for the case of sniffers appears to be the following. In the absence of a precise value of the parameters of the medium, one can start searches with rough estimates which ensure that the rate function $R(r|r_0)$ is shallower than in reality. The purpose is that, in the absence of precise information, broad estimators of the rate function will avoid wrong estimates that could drive the searcher astray e.g., to the boundaries of the search space where the search is taking place. The searcher will thus typically arrive to the source, albeit in times much longer than with the correct estimation of $R(r|r_0)$. Once arrived to the source, and thus knowing the source position $r_0$, the searcher can use the trace T of detections along its trajectory to estimate the parameters of the medium and the source.

For example, let us consider the problem of learning parameters for the model described previously. Suppose that the mean velocity V is accessible via the sensors and, just for the sake of the argument, that the turbulent diffusivity D, the RMS velocity fluctuations $V_{rms}$ and the particle lifetime $\tau$ are related as $D = V_{rms}^2 \tau$ . This is a classical dimensional estimate of turbulent diffusivity and reduces the space of parameters to two variables so that one can visualize the likelihood surface. One shall take the rate of emission of the source R and the effective diffusivity D as independent variables and try to learn the parameters of a medium having unit values for D, R and V and a turbulence level $V_{rms}/V$ = 20%, a typical value in turbulent jets. Choosing a shallow rate function $R(r|r_0)$ as initial guess corresponds to safe overestimates of D and 1/R e.g., by two orders of magnitude $D_{est}$ = 1/$R_{est}$ = 100. Starting the searcher with these very rough estimates and from initial distances 50 i.e., twice the advection length $V\tau$ = 25, slows down the search by about a factor 7. Still, more than half of the searches arrive to the source without ever touching the boundaries of the search space. The minimum is clearly located at the real values D = R = 1 and the convexity of the curve makes it easy to find it by any standard minimization algorithms, such as simplex method, simulated annealing or conjugate gradient.

Estimation of the posterior probability distribution

In the following description, $\tau_t$ denotes times and coordinates of the "hits" i.e., the points of the robot trace where sensors have been activated. The trace $\tau_t$ might be thought of as a message, sent by the particle or molecule source and transmitted to the searcher with strong noise due to the random nature of particle or molecule propagation in the turbulent medium. Decoding of the message is implemented using Bayes formula to construct, given the received signal, the posterior probability distribution $P_t(r_0)$ for the unknown location of the source $r_0$. The trace $\tau_t$ and the posterior probability distribution $P_t(r_0)$ are dynamical objects, continuously updated with time. The specific decoding protocol depends of course on the nature of the detection events and the transmitting medium. For sake of illustration, the evaluation of the probability distribution $P_t(r_0)$ is based upon the particle or molecule diffusion model discussed above.

The probability distribution posterior to experiencing a trace $\tau_t$ of uncorrelated odor encounters can be expressed as follow,

$$P_t(r_0) = \frac{\varsigma_{r_0}(\tau_t)}{\int \varsigma_x(\tau_t)dx} = \frac{\exp\left[-\int_0^t R(r(t')|\,\mathrm{r}_0)dt'\right]\prod_{i=1}^{H} R(r(t_i)|\,\mathrm{r}_0)}{\int \exp\left[-\int_0^t R(r(t')|\,x)dt'\right]\prod_{i=1}^{H} R(r(t_i)|\,x)dx} \qquad (10)$$

where, H is the number of hits along the trajectory, the $t_i$'s are the corresponding times and $\zeta r_0(\tau_t)$ is the likelihood to observe the trace $\tau_t$ of odor encounters for a source located at $r_0$. This expression is supplemented by the prescription that visited regions where the source was not found have zero probability. Note that $P_{t+\Delta t}(r_0)$ factorizes as $P_t(r_0)$ times a term that depends on the hits received in the $\Delta t$ interval. Thus, keeping track of the whole trajectory and history of detections is not required. The expression for $P_t(r_0)$ is derived by taking the probability of a "hit" during an infinitesimal interval dt to be $R(r|r_0)dt$ and the probability of not being hit to be $\exp[-R(r|r_0)dt]$.

There are obvious quantitative improvements one could obtain by accounting for correlations in the detections within coherent plumes. Detailed models of plumes have already been discussed in the literature and thus, a brief discussion of a simple way to account for time-correlations in the detections is presented here. The model is based on the following likelihood of experiencing a trace $\tau_t$ of correlated odor encounters,

$$\varsigma_{r_0}(\tau_t) = e^{-\sum_i \left[\int_{V_i} R(r(t')|\mathrm{r}_0)dt' + \int_{D_i} Q(r(t')|\mathrm{r}_0)dt'\right]} \prod_{i=1}^{H} R(r(t_i)|\,\mathrm{r}_0)\prod_{j=1}^{H'} Q(r(t_j)|\,\mathrm{r}_0) \qquad (11)$$

wherein, the $V_i$'s ($D_i$'s) are the time intervals of absence (presence) of detections, H and the $t_i$'s are the number and times of transition from no-detection to detection intervals ($V_i \to D_i$) and, finally, H' and $t'_i$ are the number and times of the opposite transitions, from detection to no-detection. The function $Q(r(t_j)|r_0)$ controls the extension of patches of particles or molecules as a function of the distance to the source $r_0$. The posterior probability distribution $P_t(r_0)$ is constructed as done previously, by using the Bayes formula,

$$P_t(r_0) = \frac{\xi_{r_0}(\tau_t)}{\int \xi_x(\tau_t)dx} \qquad (12)$$

The following expression amounts to assuming that patches of odors have finite extensions and thus, the searcher will spend finite amounts of time within them. As mentioned above, the extension of patches as a function of the distance to the source is controlled by the function $Q(r|r_0)$. In the simplest possible setting where the function $Q(r|r_0)$ is taken to be constant in space, the expression for the posterior probability distribution simplifies as,

$$P_t(r_0) = \frac{\exp\left[-\sum_i \int_{V_y} R(r(t')|\,\mathrm{r}_0)dt'\right]\prod_{i=1}^{H} R(r(t_i)|\,\mathrm{r}_0)}{\int \exp\left[-\sum_i \int_{V_y} R(r(t')|\,x)dt'\right]\prod_{i=1}^{H} R(r(t_i)|\,x)dx} \qquad (13)$$

The same structure as for independent hits is obtained but consecutive detections are not overcounted. One should notice that there are no additional parameters to be estimated. Variations along the same lines might be considered by introducing additional parameters, for example treating the time distance to a previous hit in a patch as a free parameter and estimating it from the data.

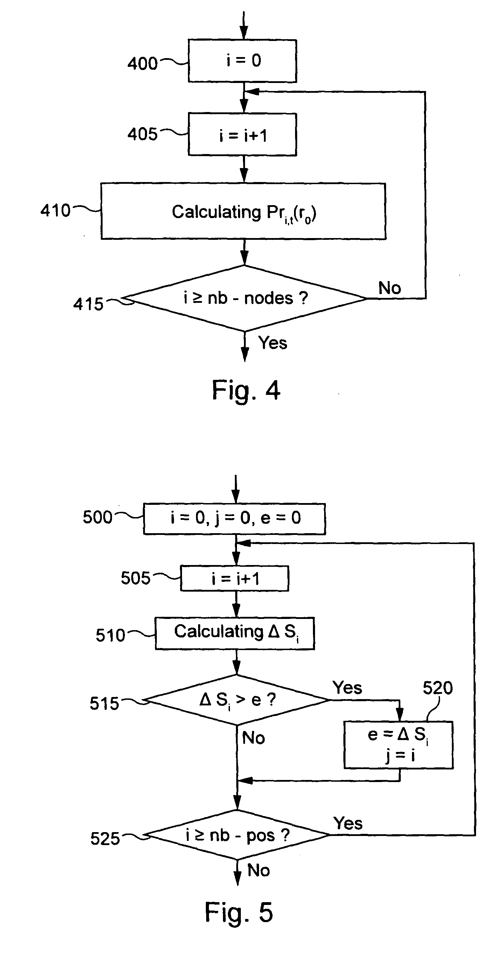

Figure 4 depicts the main steps of evaluating the posterior probability of each node of the search space lattice, corresponding to box 315 of figure 3. As illustrated, a variable i representing the index of a node of the searched space lattice among all the nodes is set to zero (step 400) and incremented by one (step 405). The probability $P_{r_{i,t}}(r_0)$ that node $r_i$ corresponds to the source $r_0$ is calculated according to relation 10 or to relation 13 (step 410) and a test is performed to determine whether or not the probability of each node of the search space lattice has been evaluated at time t (step 415). If the probability of all the nodes of the search space lattice have not been evaluated at time t the last three steps i.e., steps 405 to 415, are repeated.

Determination of the sensor movement

Given a probability distribution $P(r_0)$ for the location of the source, the Shannon's entropy S for the distribution can be expressed as,

$$S \equiv -\int dx P(x) \ln P(x) \qquad (14)$$

The entropy quantifies how spread-out the distribution is and goes to zero when the position of the source is localized to one site i.e., is known. The rate of acquisition of information is quantified by the entropy rate of reduction. The main problem for the searcher is that the real probability distribution is unknown (to it) and must be estimated from the available data i.e., the history of its odor encounters. As information accumulates, the entropy of the estimated distribution decreases and with it the expected time to locate the source. The searcher is faced with conflicting choices of either proceeding with its current information i.e., going to the estimated most probable source location, or alternatively, pausing to gather more information and obtain a more reliable estimate of the source distribution. The problem of dealing with only partially reliable information is quite general and has received a systematic formulation in learning theory in terms of the "exploration versus exploitation tradeoff" to be struck for effective learning. In the search context, "exploitation" of the currently estimated $P_t(r_0)$ by chasing locations of maximal estimated probability is very risky because it can lead off the track. The most conservative "exploration" approach is to accumulate information before taking any step. This strategy is safe but not productive and is inferior to more active exploration, e.g. systematic search in a particular sector.

According to the present invention, the searcher chooses, at each time step, the direction which locally maximizes the expected rate of information acquisition, to balance exploration and exploitation. Specifically, the searcher chooses, among the neighbouring nodes of the lattice and standing still, the movement which maximizes the expected reduction in entropy of the posterior probability field. Expectations are based on the information currently available i.e., the field $P_t(r_0)$ itself. Entropy decreases (and thus information accumulates) faster close to the source because cues arrive at a higher rate, hence tracking maximum rate of information acquisition will guide to the source much like concentration gradients in chemotaxis.

Supposing that the searcher has arrived at r at time t and gathered information is stored into the field $P_t(r_0)$ having entropy S, the variation of entropy expected upon moving to one of the neighbouring nodes $r_j$ (or standing still) is expressed as,

$$\overline{\Delta S}(r \to r_j) = -SP_t(r_j) + (1 - P_t(r_j))[\rho_0(r_j)\Delta S_0 + \rho_1(r_j)\Delta S_1 + \cdots + \rho_n(r_n)\Delta S_n] \qquad (15)$$

The first term on the right-hand side corresponds to finding the source i.e., $P_{t+1}$ becoming a δ-function and entropy becoming zero, which occurs with estimated probability $P_t(r_j)$. The second term corresponds to the alternative case when the source is not found at $r_j$. Symbols $\rho_k(r_j)$ denote the probability that k detections be made at $r_j$ during a time step $\Delta t$, given, for independent detections, by a Poisson law,

$$\rho_k \equiv \frac{h^k e^{-h}}{k!} \qquad (16)$$

The expected number of hits is estimated as h($r_j$), where,

$$h(r_j) \equiv \Delta t \int P_t(r_0) R(r_j \mid r_0) dr_0 \qquad (17)$$

with R($r|r_0$) denoting the mean rate of hits at position r if the source is located in $r_0$, as mentioned above.

The symbols $\Delta S_k$ denote the change of entropy between the fields $P_{t+1}(r_0)$ and $P_t(r_0)$. As derivable from relation (15), two effects contribute to $\Delta S_k$,

- $P_{t+1}(r_j) \equiv 0$ since the source was not found; and,
- the estimated posterior probabilities are modified by the k cues received.

The first term in is the exploitative term, weighing only the event that the source is found at the point $r_j$ and favouring motion to maximum likelihood points. The second contribution is the information gain from receiving additional cues. It appears even when the searcher does not move and thus represents conservative "exploration". Thus, it is observable that the algorithm according to the invention naturally combines both exploitative and exploratory strategies.

Therefore, after having estimated the posterior probabilities of all the nodes of the lattice, the entropy variation of each of the next possible sensor position is evaluated so as to choose the one that maximise the entropy reduction. Figure 5 shows the main steps of evaluating the next optimal movement of the particle or molecule sensor according to the posterior probability of each node of the search space lattice, corresponding to box 320 of figure 3. After variables i, j, and e have been initialized to zero (step 500), variable i, representing the possible moves of the sensor, is incremented by one (step 505). The variation of entropy $\overline{\Delta S}(r \to r_i)$ of the probability field $P(r_0)$ is calculated according to relation 15 (step 510) and a test is performed to determine whether or not the variation of entropy $\overline{\Delta S}(r \to r_i)$ is greater than variable e (step 515). If the variation of entropy $\overline{\Delta S}(r \to r_i)$ is greater than variable e, variable e is set to the value of the variation of entropy $\overline{\Delta S}(r \to r_i)$ and variable j is set to the value of variable i (step 520). Then, a second test is performed to determine whether or not the variation of entropy $\overline{\Delta S}(r \to r_i)$ has been calculated for all the possible moves of the sensor (step 525). If the variation of entropy $\overline{\Delta S}(r \to r_i)$ has not been calculated for all the possible moves of the sensor, the last four steps i.e., steps 505 to 525, are repeated. At the end of the process, variable j indicates the sensor movement that maximizes the variation of entropy $\overline{\Delta S}(r \to r_i)$.

Search time

The aim of this section is to derive a lower bound on the expected search time T as a function of the entropy S of the probability distribution for the source location. To that aim, one shall consider the ensemble of probability distributions with fixed entropy S and compute the expected minimal time. In obtaining a lower bound one can relax continuity constraints and allow the searcher to jump, like a grasshopper, from one site to any other. For simplicity, here is considered a spatial lattice with unit mesh size, set by the linear dimension of the searcher which one can choose as a unit of length. Denoting by $p_j$ the probability to find the source at the j-th (in time) point visited, the expected search time reads $T = \sum_j j p_j$. The best possible option is to manifestly visit points in decreasing order of probability and the desired lower bound is obtained by minimization with respect to all possible distributions $p_j$ with fixed entropy S. Thus, one should minimize the following relation,

$$T' = \sum_j j p_j + \alpha \left( \sum_j p_j - 1 \right) + \beta^{-1} \left( \sum_j p_j \log p_j + S \right) \qquad (18)$$

where $\alpha$ and $\beta$ are the Lagrange multipliers enforcing the normalization of probability and the constraint on the entropy. The probability distribution corresponding to the minimum of the previous relation has a Gibbs form: $p_j \propto \exp(-\beta j)$. If boundaries can be neglected i.e., S<<logN where N is the total number of points, the inverse temperature $\beta$ is such that $\beta$>>1/N. The relation between the entropy and the search time reads then,

$$S = T \log T - (T - 1)\log(T - 1) \tag{19}$$

In the interesting cases where S and T are both large compared to unity, this reduces to the following relation,

$$T = \sum_j j p_j \approx e^{S-1} \equiv \frac{\bar{n}}{e} \tag{20}$$

In other words, the search time is bounded in terms of the effective number of points $n = e^s$, which is determined by entropy.

Simulation

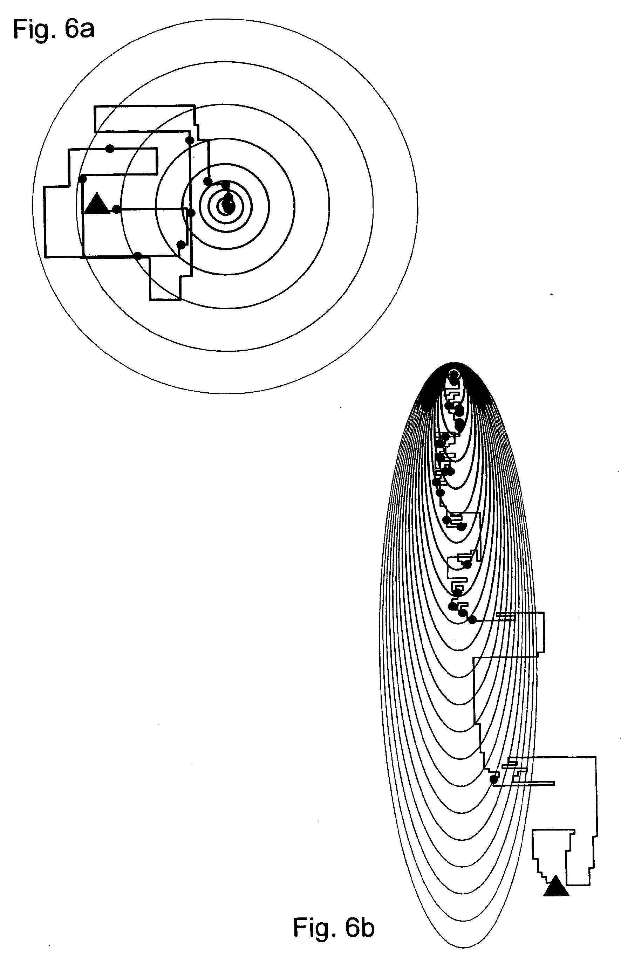

Figure 6, comprising figures 6a and 6b, presents the result of a numerical simulation using a model of odor propagation described above, in the absence of wind and in the presence of wind, respectively. Simulations of the search process are realized using a computational model of odor spreading where detectable particles or molecules are emitted by the source at rate R, have a finite lifetime $\tau$, propagate with isotropic effective diffusivity D (which parameterizes the combined effect of turbulent and molecular diffusion) and are advected by a mean current or wind V. This simple model provides a reasonable representation of turbulent advection and mixing. The background concentric lines represent the mean rate of particle or molecule detections and in both cases the rate decays exponentially at large distances. Searcher starts from the points indicated by black upward triangles. Odor detection events along the trajectories are indicated by black circles. Note the long lags where no particles are detected, reflecting the dilute conditions characteristic of odor detection at large distances. In the absence of wind (figure 6a), symmetry around the starting point is initially present and the searcher starts spiralling around it (as is observed with sea urchin sperm). Interestingly, in the absence of hits, the radius of the spiral increases in a scale invariant manner, making an approximately Archimedean spiral. As time progresses and information is gathered along the trajectory, the symmetry is broken more and more until the direction towards the source emerges as the preferential one, finally leading thereto. In the presence of wind (figure 6b), the search alternates phases of consistent progression upwind with phases of wider crosswind excursion and even downwind movements suggestive of the classical casting and zigzagging patterns observed during bird and moth flights.

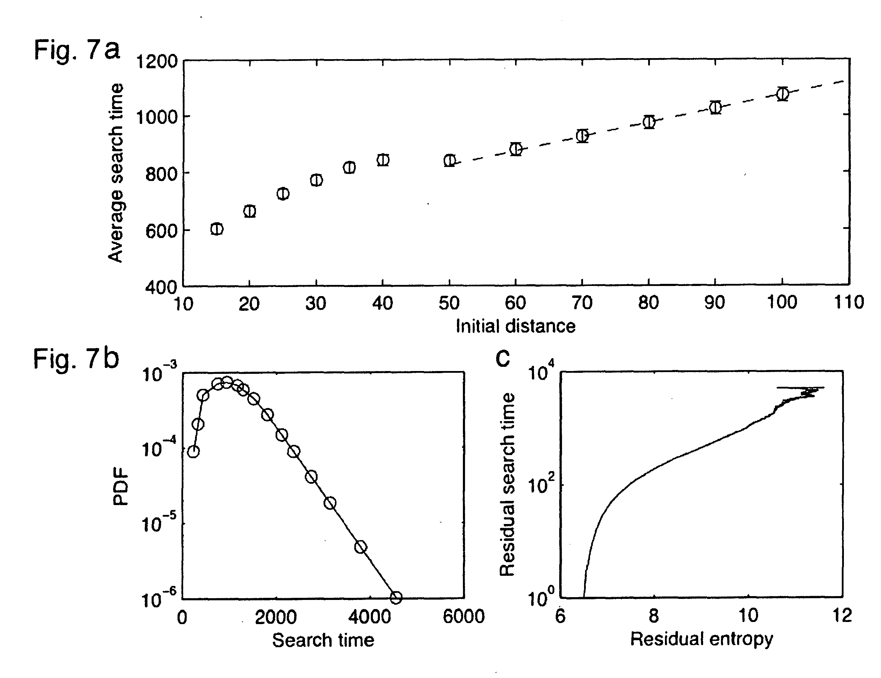

Figure 7 depicts the quantitative characterization of search algorithm. Figure 7a shows the scaling of the average search time with respect to the initial distance to the source. The mean path travelled by particles or molecule during their lifetime is 50, corresponding to the plateau observed in the figure. The linear scaling at large initial distances favourably compares with the exponential time needed to average out concentration noise. Figure 7b represents the typical exponential decay of the probability distribution function (PDF) of the search times, indicating that searches are not plagued by strong fluctuations. Figure 7c is the scaling of the residual search time (time left to locate the source) versus the entropy of the estimated probability distribution for the source location. The quantities are averaged over an ensemble of 1,000 realizations of the search process. Note the exponential dependence of the search time on the entropy of the field, in agreement with theoretical arguments. The exponential dependence indicates that reducing entropy in the estimation of the source location is an effective way to ensure a rapid search process.

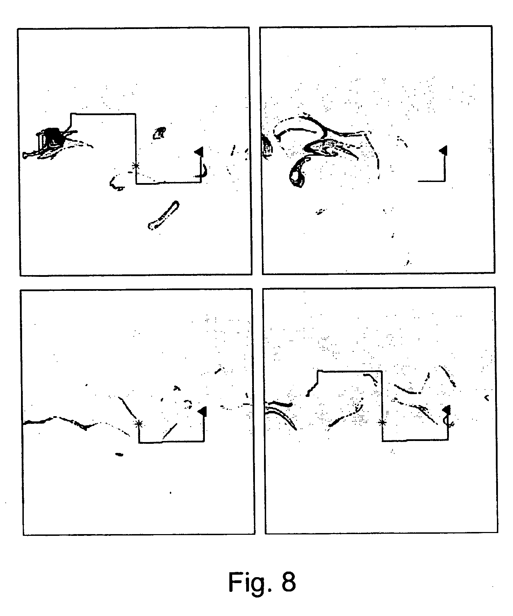

Figure 8 presents the path generated in a simulation utilizing experimental measurements of dye concentration in a turbulent flow. "Hits" occur when the searcher encounters concentration above a threshold, which we chose sufficiently high to keep the number of hits low. Simulations indicate that the strategy according to the invention is robust with respect to the searcher's model of the turbulent medium and to fluctuations and inhomogeneities of the medium. Indeed, even the simplistic hypothesis of time-independent odor encounters does not hinder the search. Modelling of the turbulent medium might be further improved by accounting for temporal correlations of odor plume encounter.

Naturally, in order to satisfy local and specific requirements, a person skilled in the art may apply to the solution described above many modifications and alterations all of which, however, are included within the scope of protection of the invention as defined by the following claims.

**Claims**

**1.** A method for locating a source of particles, molecules, or fragments of molecules in a search space using a mobile sensor adapted to detect the presence of such particle, molecule, or fragment of particle, the method comprising the steps of,

- determining (300) the particle, molecule, or fragment of molecule diffusion parameters in said search space;
- designing (305) a lattice on said search space;
- determining (310) if at least one particle, molecule, or fragment of molecule is detected by said sensor;
- computing (315) a probability for each node of said search space lattice to be the source of said particles, molecules, or fragments of molecules according to the determined particle, molecule, or fragment of molecule diffusion parameters and to the detected particles, molecules, or fragments of molecules;
- evaluating (320) a movement of said sensor according to said computed probabilities.

2.  The method of claim 1 further comprising the step of repeating, until one of said computed probabilities is approximately equal to a predetermined threshold, the steps of,

- determining if at least one particle, molecule, or fragment of molecule is detected by said sensor;
- computing a probability for each node of said search space lattice to be the source of said particle, molecule, or fragment of molecule according to the determined particle, molecule, or fragment of molecule diffusion parameters and to the detected particles, molecules or fragments of molecules;
- evaluating a movement of said sensor according to said computed probabilities; and,
- moving said sensor.

3.  The method of either claim 1 or claim 2 wherein the rate $R(r|r_0)$ at which particles, molecules, or fragments of molecules are received at point r from the source of said particles, molecules, or fragments of molecules located in $r_0$ is expressed as follow,

$$R(r \mid r_0) = \frac{aR}{|r - r_0|} e^{\frac{-(r-r_0)}{\lambda}} e^{\frac{(y_0 - y)V}{2D}}$$

with

$$\lambda = \sqrt{\frac{D\tau}{1 + \frac{V^2 \tau}{4D}}}$$

the particle, molecule, or fragment of molecule diffusion parameters being,
a that represents the particle, molecule, or fragment of molecule radius;
R that is the particle, molecule, or fragment of molecule emission rate;
$\tau$ that represents the particle, molecule, or fragment of molecule lifetime;
V that is the mean flow presents in said search space;
$(y_0 - y)$ that characterizes the mean flow direction; and,
D that represents the diffusivity of the particles, molecules, or fragments of molecules.

4.  The method of any one of claims 1 to 3 wherein said probability $P_t(r_0)$ associated to each node of said search space lattice to be the source of said particles, molecules, or fragments of molecules is computed according to the next relation,

$$P_t(r_0) = \frac{\exp\left[-\sum_i \int_{V_y} R(r(t')\,|\,r_0)\,dt'\right]\prod_{i=1}^{H} R(r(t_i)\,|\,r_0)}{\int \exp\left[-\sum_i \int_{V_y} R(r(t')\,|\,x)\,dt'\right]\prod_{i=1}^{H} R(r(t_i)\,|\,x)\,dx}$$

where $R(r|r_0)$ is the rate at which particles, molecules, or fragments of molecules are received at point r from the source of said particles, molecules, or fragments of molecules located in $r_0$.

5.  The method of any one of the previous claims wherein said step of evaluating a movement of said sensor according to said computed probabilities is based upon the entropy of said computed probabilities.

6.  The method of claim 5 wherein the movement of said sensor is determined so as to maximize the variation of entropy of said computed probabilities.

7.  The method of claim 6 wherein the variation $\overline{\Delta S}(r \to r_j)$ of entropy S of said computed probabilities is determined according to the following relation,

$$\overline{\Delta S}(r \to r_j) = -SP_t(r_j) + (1 - P_t(r_j))[\rho_0(r_j)\Delta S_0 + \rho_1(r_j)\Delta S_1 + \cdots + \rho_n(r_n)\Delta S_n]$$

wherein,
$P_t(r_j)$ is the probability at time t that node $r_j$ corresponds to the source of said particles, molecules, or fragments of molecules;
$\rho_i(r_j)$ denotes the probability that i particle, molecule, or fragment of molecule detections are made at node $r_j$ during a time step $\Delta t$; and,
$\Delta S_i$ denotes the change of entropy between the fields $P_{t+1}(r_0)$ and $P_t(r_0)$.

8.  The method of any one of the previous claims wherein at least two different types of particles, molecules, or fragments of molecules are emitted by said source of particles, molecules, or fragments of molecules to be localized, said mobile sensor being adapted to detect each of said at least two different types of particles, molecules, or fragments of molecules, a probability being computed for each of said at least two different types of particles, molecules, or fragments of molecules, for each node of said search space lattice to be the source of said particles, molecules, or fragments of molecules.

9.  The method of any one of the previous claims wherein said molecules or fragments of molecules are biological molecules, biological fragments of molecules, proteins, fragments of proteins, or pathogens.

10. The method of any one of the previous claims wherein said particles, molecules, or fragments of molecules characterizes an odorous substance.

11. The method of any one of the previous claims wherein said search space is located within a gaseous environment or within a liquid environment.

12. An apparatus comprising means adapted for carrying out each step of the method according to any one of the claims 1 to 11.

13. The apparatus of claim 12 comprising first means embedding said sensor, said first means being mobile in said search space, and second means for computing said probabilities and evaluating said movement, said first and second means comprising means for exchanging data.

14. A computer-like readable medium comprising instructions for carrying out each step of the method according to any one of the claims 1 to 11.

**Patentansprüche**

1. Verfahren zum Auffinden einer Quelle von Teilchen, Molekülen oder Fragmenten von Molekülen in einem Suchraum mittels eines mobiler Sensors, der ausgerüstet ist, die Anwesenheit von derartigen Teilchen, Molekülen oder Fragmenten von Teilchen oder Molekülen aufzufinden, wobei das Verfahren die Schritte aufweist, der

   - Bestimmung (300) der Diffusionsparameter der Teilchen, Moleküle oder Fragmente der Moleküle in dem Suchraum;
   - Bestimmung (305) einer Gitterstruktur im Suchraum;
   - Bestimmung (310), ob zumindest ein Teilchen, Molekül oder Fragment eines Moleküls durch den Sensor erkannt wurde;
   - Berechnung (315) der Wahrscheinlichkeit für jeden Knoten der Suchraumgitterstruktur eine Quelle der Teilchen, Moleküle oder Fragmente der Moleküle zu sein in Abhängigkeit der bestimmten Diffusionsparameter der Teilchen, Moleküle oder Fragmente der Moleküle und der aufgefundenen Teilchen, Moleküle oder Fragmente der Moleküle;
   - Bestimmung (320) einer Bewegung des Sensors in Abhängigkeit der berechneten Wahrscheinlichkeiten.

2. Verfahren nach Anspruch 1, welches zusätzlich den Schritt einer Wiederholung aufweist, solange bis die berechnete Wahrscheinlichkeit annähernd gleich zu einem vorbestimmten Schwellwert ist, mit den Schritten der

   - Bestimmung, ob zumindest ein Teilchen, Molekül oder Fragment von Molekülen durch den Sensor erkannt wurde;
   - Berechnung (315) der Wahrscheinlichkeit für jeden Knoten der Suchraumgitterstruktur eine Quelle der Teilchen, Moleküle oder Fragmente der Moleküle zu sein in Abhängigkeit der bestimmten Diffusionsparameter der Teilchen, Moleküle oder Fragmente der Moleküle und der aufgefundenen Teilchen, Moleküle oder Fragmente der Moleküle;
   - Bestimmung (320) einer Bewegung des Sensors in Abhängigkeit der berechneten Wahrscheinlichkeiten; und
   - Bewegung des Sensors.

3. Verfahren nach entweder Anspruch 1 oder Anspruch 2, wobei die Menge $R(r \mid r_0)$ mit der die Teilchen, Moleküle oder Fragmente der Moleküle am Punkt r von der Quelle der Teilchen, Moleküle oder Fragmente der Moleküle empfangen werden, wie folgt ausgedrückt wird:

$$R(r \mid r_0) = \frac{aR}{|r - r_0|} e^{\frac{-(r-r_0)}{\lambda}} e^{\frac{(y_0-y)V}{2D}}$$

mit:

$$\lambda = \sqrt{\frac{D\tau}{1 + \frac{V^2\tau}{4D}}}$$

und die Parameter der Teilchen, Moleküle oder Fragmente der Moleküle sind:

   a ist der Radius der Teilchen, Moleküle oder Fragmente der Moleküle,
   R ist die Emissionsrate der Teilchen, Moleküle oder Fragmente der Moleküle,
   $\tau$ ist die Lebensdauer der Teilchen, Moleküle oder Fragmente der Moleküle,
   V ist der Durchschnittsfluss im genannten Suchraum,
   $(y_0\text{-}y)$ ist die mittlere Fließrichtung und
   D für das Diffusionsvermögen der Teilchen, Moleküle oder Fragmente der Moleküle steht.

4. Verfahren nach einem der Ansprüche 1 bis 3, wobei die Wahrscheinlichkeit $P_t(r_0)$ jedem Knoten der Suchraumgitterstruktur zugeordnet ist, die Quelle der Teilchen, Moleküle oder Fragmente der Moleküle zu sein, mit folgender Formel berechnet wird,

$$P_t(r_0) = \frac{\exp\left[-\sum_i \int_{V_r} R(r(t') \mid r_0)dt'\right]\prod_{i=1}^{H} R(r(t_i) \mid r_0)}{\int \exp\left[-\sum_i \int_{V_r} R(r(t') \mid x)dt'\right]\prod_{i=1}^{H} R(r(t_i) \mid x)dx}$$

wobei $R(r \mid r_0)$ die Menge am Punkt r ist, mit der die Teilchen, Moleküle oder Fragmente der Moleküle von der Quelle der Teilchen, Moleküle oder Fragmente der Moleküle erhalten werden, und die Quelle bei $r_o$ ist.

5. Verfahren nach einem der vorangegangenen Ansprüche, wobei der Schritt der Berechung der Bewegung des Sensors mithilfe der berechneten Wahrscheinlichkeit auf der Entropie der berechneten Wahrscheinlichkeiten basiert.

6. Verfahren nach Anspruch 5, wobei die Bewegung des Sensors **dadurch** bestimmt wird, das die Abweichung der Entropie der berechneten Wahrscheinlichkeiten maximiert wird.

7. Verfahren nach Anspruch 6, wobei die Unterschiede $\overline{\Delta S}(r \to r_j)$ der Entropie S der berechneten Wahrscheinlichkeiten über folgende Beziehung bestimmt wird:

$$\overline{\Delta S}(r \to r_j) = -SP_t(r_j) + (1 - P_t(r_j))[\rho_0(r_j)\Delta S_0 + \rho_1(r_j)\Delta S_1 + \cdots + \rho_n(r_n)\Delta S_n]$$

wobei
$P_t(r_j)$ die Wahrscheinlichkeit zur Zeit t ist, dass der Knoten $r_j$ der Quelle der Teilchen, Moleküle oder Fragmente der Moleküle entspricht,
$\rho_i(r_j)$ die Wahrscheinlichkeit bestimmt, dass i Bestimmungen der Teilchen, Moleküle oder Fragmente der Moleküle am Knoten $r_j$ während eines Zeitschritts $\Delta t$ durchgeführt werden; und
$\Delta S_i$ die Veränderung der Entropie zwischen den Feldern $P_{t+1}(r_0)$ und $P_t(r_0)$ bestimmt.

8. Verfahren nach einem der vorangegangenen Ansprüche, wobei zumindest zwei unterschiedliche Arten von Teilchen, Moleküle oder Fragmente der Moleküle von der zu bestimmenden Quelle der Teilchen, Moleküle oder Fragmente der Moleküle emittiert werden und der mobile Sensor geeignet ist, jedes der zumindest zwei unterschiedlichen Arten der Teilchen, Moleküle oder Fragmente der Moleküle zu erkennen, eine Wahrscheinlichkeit für jeden Knoten der Suchraumgitterstruktur und für jedes der zumindest zwei unterschiedlichen Teilchen, Moleküle oder Fragmente der Moleküle berechnet wird, die Quelle der Teilchen, Moleküle oder Fragmente der Moleküle zu sein.

9. Verfahren nach einem der vorangegangenen Ansprüche, wobei die Teilchen, Moleküle oder Fragmente der Moleküle biologische Moleküle, biologische Fragmente der Moleküle, Eiweiße, Fragmente von Eiweißen oder Krankheitserreger sind.

10. Verfahren nach einem der vorangegangenen Ansprüche wobei die Teilchen, Moleküle oder Fragmente der Moleküle eine riechende Substanz bestimmen.

11. Verfahren nach einem der vorangegangenen Ansprüche wobei der Suchraum innerhalb einer gasförmigen oder einer flüssigen Umgebung vorgesehen ist.

12. Gerät mit Vorrichtungen geeignet ein Verfahren nach einem der Ansprüche 1 bis 11 durchzuführen.

13. Gerät gemäß Anspruch 12, wobei erste Mittel den Sensor umfassen, die ersten Mittel in dem Suchraum beweglich sind und zweite Mittel zum Berechnen der Wahrscheinlichkeiten und der Bestimmung der Bewegung vorgesehen sind und die ersten und zweiten Mittel Informationsaustauschmittel aufweisen.

**14.** Medium, durch einen Computer lesbar, welches Anweisungen enthält ein Verfahren nach einem der Ansprüche 1 bis 11 durchzuführen.

**Revendications**

**1.** Procédé de localisation d'une source de particules, de molécules ou de fragments de molécules dans un espace de recherche en utilisant un capteur mobile apte à détecter la présence de cette particule, de cette molécule ou de ce fragment de particule, le procédé comprenant les étapes consistant à :

- déterminer (300) les paramètres de diffusion de la particule, de la molécule ou du fragment de molécule dans ledit espace de recherche ;
- créer (305) un maillage sur ledit espace de recherche ;
- déterminer (310) si au moins une particule, une molécule ou un fragment de molécule est détecté par ledit capteur ;
- calculer (315) une probabilité pour chaque noeud dudit maillage de l'espace de recherche d'être la source desdites particules, desdites molécules ou desdits fragments de molécules conformément aux paramètres de diffusion déterminés des particules, des molécules ou des fragments de molécules et aux particules, aux molécules ou aux fragments de molécules détectés ;
- évaluer (320) un mouvement dudit capteur en fonction desdites probabilités calculées.

**2.** Procédé selon la revendication 1, comprenant en outre l'étape consistant à répéter, jusqu'à ce que l'une desdites probabilités calculées soit approximativement égale à un seuil prédéterminé, les étapes consistant à :

- déterminer si au moins une particule, une molécule ou un fragment de molécule est détecté par ledit capteur ;
- calculer une probabilité pour chaque noeud dudit maillage de l'espace de recherche d'être la source de ladite particule, de ladite molécule ou dudit fragment de molécule conformément aux paramètres de diffusion déterminés des particules, des molécules ou des fragments de molécules et aux particules, aux molécules ou aux fragments de molécules détectés ;
- évaluer un mouvement dudit capteur en fonction desdites probabilités calculées ; et
- déplacer ledit capteur.

**3.** Procédé selon l'une quelconque des revendications 1 et 2, dans lequel le taux $R(r|r_0)$ de réception de particules, de molécules ou de fragments de molécules en un point r en provenance de la source desdites particules, desdites molécules ou desdits fragments de molécules située en $r_0$, s'exprime de la manière suivante :

$$R\left(r \mid r_0\right) = \frac{aR}{\left|r - r_0\right|} e^{\frac{-(r-r_0)}{\lambda}} e^{\frac{(y_0-y)V}{2D}}$$

avec

$$\lambda = \sqrt{\frac{D\tau}{1 + \frac{V^2\tau}{4D}}}$$

les paramètres de diffusion des particules, des molécules ou des fragments de molécules étant :

a, qui représente le rayon de la particule, de la molécule ou du fragment de molécule ; R, qui représente le taux d'émission de la particule, de la molécule ou du fragment de molécule ;

$\tau$, qui représente la durée de vie de la particule, de la molécule ou du fragment de molécule ;

V, qui est le flux moyen présent dans ledit espace de recherche ;

$(y_0\text{-}y)$, qui caractérise la direction du flux moyen ; et

D, qui représente la diffusivité des particules, des molécules ou des fragments de molécules.

**4.** Procédé selon l'une quelconque des revendications 1 à 3, dans lequel ladite probabilité $P_t(r_0)$ associée à chaque noeud dudit maillage de l'espace de recherche d'être la source desdites particules, desdites molécules ou desdits fragments de molécules, est calculée conformément à la relation suivante :

$$P_t(r_0) = \frac{\exp\left[-\sum_i \int_{V_y} R(r(t')|r_0)dt'\right] \prod_{i=1}^{H} R(r(t_i)|r_0)}{\int \exp\left[-\sum_i \int_{V_y} R(r(t')|x)dt'\right] \prod_{i=1}^{H} R(r(t_i)|x)dx}$$

où $R(r|r_0)$ est le taux de réception de particules, de molécules ou de fragments de molécules en un point r en provenance de la source desdites particules, desdites molécules ou desdits fragments de molécules située en $r_0$.

**5.** Procédé selon l'une quelconque des revendications précédentes, dans lequel ladite étape consistant à évaluer un mouvement dudit capteur en fonction desdites probabilités calculées est fondée sur l'entropie desdites probabilités calculées.

**6.** Procédé selon la revendication 5, dans lequel le mouvement dudit capteur est déterminé de façon à rendre maximale la variation d'entropie desdites probabilités calculées.

**7.** Procédé selon la revendication 6, dans lequel la variation $\overline{\Delta S}(r \rightarrow r_j)$ de l'entropie S desdites probabilités calculées est déterminée conformément à la relation suivante :

$$\overline{\Delta S}(r \rightarrow r_j) = -S P_t(r_j) + (1 - P_t(r_j))\left[\rho_0(r_j)\Delta S_0 + \rho_1(r_j)\Delta S_1 + \ldots + \rho_n(r_n)\Delta S_n\right]$$

où
$P_t(r_j)$ est la probabilité, au temps t, que le noeud $r_j$ corresponde à la source desdites particules, desdites molécules ou desdits fragments de molécules ;
$\rho_i(r_j)$ désigne la probabilité que i détections de particules, molécules ou fragments de molécules soient effectuées au noeud $r_j$ pendant un pas temporel de $\Delta t$ ; et
$\Delta S_i$ désigne la variation d'entropie entre les champs $P_{t+1}(r_0)$ et $P_t(r_0)$.

**8.** Procédé selon l'une quelconque des revendications précédentes, dans lequel au moins deux types différents de particules, de molécules ou de fragments de molécules sont émis par ladite source de particules, de molécules ou de fragments de molécules devant être localisée, ledit capteur mobile étant apte à détecter chacun desdits au moins deux types différents de particules, de molécules ou de fragments de molécules, une probabilité étant calculée, pour chacun desdits au moins deux types différents de particules, de molécules ou de fragments de molécules, pour chaque noeud dudit maillage de l'espace de recherche d'être la source desdites particules, desdites molécules ou desdits fragments de molécules.

**9.** Procédé selon l'une quelconque des revendications précédentes, dans lequel lesdites molécules ou lesdits fragments de molécules sont des molécules biologiques, des fragments de molécules biologiques, des protéines, des fragments de protéines ou des agents pathogènes.

**10.** Procédé selon l'une quelconque des revendications précédentes, dans lequel lesdites particules, lesdites molécules ou lesdits fragments de molécules caractérisent une substance odorante.

**EP 2 041 636 B1**

**11.** Procédé selon l'une quelconque des revendications précédentes, selon lequel ledit espace de recherche est situé dans un environnement gazeux ou dans un environnement liquide.

**12.** Appareil comprenant des moyens aptes à mettre en oeuvre chaque étape du procédé selon l'une quelconque des revendications 1 à 11.

**13.** Appareil selon la revendication 12, comprenant des premiers moyens intégrant ledit capteur, lesdits premiers moyens étant mobiles dans ledit espace de recherche, et des seconds moyens destinés à calculer lesdites probabilités et à évaluer ledit mouvement, lesdits premiers et seconds moyens comprenant des moyens permettant d'échanger des données.

**14.** Support lisible de type informatique comprenant des instructions destinées à mettre en oeuvre chaque étape du procédé selon l'une quelconque des revendications 1 à 11.

**18**

Fig. 1

Fig. 2

300 — Determining diffusion parameters

305 — Designing a lattice

310 — Detecting particles or molecules

315 — Calculating the probability field

320 — Evaluating motion direction

325 — Moving the sensor

330 — Source located or threshold reached ?          No

Yes

Fig. 3

400 — | i = 0 |

405 — | i = i+1 |

410 — | Calculating $Pr_{i,t}(r_0)$ |

415 — ⟨ i ≥ nb - nodes ? ⟩ — No

Yes

## Fig. 4

500 — | i = 0, j = 0, e = 0 |

505 — | i = i+1 |

510 — | Calculating $\Delta S_i$ |

515 — ⟨ $\Delta S_i$ > e ? ⟩ — Yes

520 — | $e = \Delta S_i$ <br> j = i |

No

525 — ⟨ i ≥ nb - pos ? ⟩ — Yes

No

## Fig. 5

Fig. 6a

Fig. 6b

Fig. 7a

Fig. 7b

c

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CAMILLI R et al.** Integrating in-situ chemical sampling with AUV control systems. *OCEANS'04. MTTS/IEEE TECHNO-OCEAN '04 KOBE,* 09 November 2004, 101-109